# FASCICULE DE BREVET EUROPEEN

(11) **EP 1 379 215 B1**
(45) Date de publication et mention de la délivrance du brevet: **16.09.2015**
(21) Numéro de dépôt: 02757747.7
(22) Date de dépôt: 29.03.2002
(51) Int. Cl.: A61Q 5/12, A61K 8/73, A61K 8/81, A61K 8/898

(54) **COMPOSITIONS COSMETIQUES CONTENANT UN PHOSPHATE D'AMIDON ET UN POLYMERE CATIONIQUE ET LEURS UTILISATIONS**
KOSMETISCHE ZUSAMMENSETZUNGEN ENTHALTEND EIN STÄRKEPHOSPHAT UND EIN KATIONISCHES POLYMER SOWIE IHRE VERWENDUNGEN
COSMETIC COMPOSITIONS CONTAINING A STARCH PHOSPHATE AND A CATIONIC POLYMER AND USES THEREOF

(30) Priorité: 30.03.2001 FR 0104387
(43) Date de publication de la demande: 14.01.2004
(73) Titulaire: L'Oréal, 75008 Paris (FR)
(72) Inventeur: FACK, Géraldine, F-92300 Levallois-Perret (FR); POURILLE-GRETHEN, Chrystel, F-92110 Clichy (FR); RESTLE, Serge, F-95390 Saint Prix (FR)
(74) Mandataire: Le Blainvaux Bellegarde, Françoise
(86) Numéro de dépôt international: PCT/FR2002/001113
(87) Numéro de publication internationale: WO 2002/078654

(56) Documents cités:
- EP-A- 0 948 958
- EP-A- 0 948 959
- WO-A-01/70270
- WO-A-98/01109
- US-B1- 6 277 893

## Description

La présente invention concerne de nouvelles compositions cosmétiques comprenant dans un milieu cosmétiquement acceptable au moins un polymère cationique non siliconé particulier, un agent tensioactif et au moins un phosphate d'amidon.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà proposé pour le traitement des matières kératiniques et en particulier des cheveux, des compositions cosmétiques contenant des polysaccharides épaississants tels que notamment l'amidon ou les celluloses.

De telles compositions présentent cependant des inconvénients tels que des problèmes de rinçabilité, des problèmes de stabilité à pH acide, des difficultés de répartition sur les matières kératiniques ainsi que des propriétés cosmétiques insuffisantes.

On a déjà préconisé dans les compositions pour le lavage ou le soin des matières kératiniques telles que les cheveux l'utilisation de polymères cationiques pour faciliter le démêlage des cheveux et pour leur communiquer douceur et souplesse. L'usage des polymères cationiques dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage.

En résumé, il s'avère que les compositions cosmétiques actuelles contenant des polymères cationiques, ne donnent pas complètement satisfaction.

WO01/70270 (appartenant à l'art antérieur selon l'art 54(3)CBE) divulgue des compositions sans tensioactif comprenant un dérivé phophorylé de l'amidon, un ou plusieurs co-épaississants et de l'eau.

WO01/01109 décrit des compositions cosmétiques comprenant dans un milieu aqueux un amidon réticulé et pré-gélatinisé éventuellement associé à une gomme de guar cationique qui n'est pas une cellulose cationique.

La demanderesse a maintenant découvert que l'association d'un phosphate d'amidon avec certains polymères cationiques, en présence d'un agent tensioactif, permet de remédier à ces inconvénients.

Ainsi, à la suite d'importantes recherches menées sur la question, il a maintenant été trouvé par la Demanderesse qu'en introduisant un phosphate d'amidon dans les compositions en particulier capillaires de l'art antérieur à base de certains polymères cationiques et en présence d'un agent tensioactif, il est possible de limiter, voire supprimer, les problèmes évoqués ci-dessus.

De plus, cette association apporte une texture fondante aux compositions cosmétiques, c'est à dire qui disparaît rapidement dans la chevelure. Les cheveux traités avec cette composition ont un toucher doux et sans résidus.

Par ailleurs, les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable, au moins un phosphate, un agent tensioactif choisi parmi les tensioactifs anioniques, non ioniques, amphotères, cationiques et leurs mélanges et au moins un polymère cationique non siliconé tel que défini ci-dessous.

Les différents objets de l'invention vont maintenant être détaillés. L'ensemble des significations et définitions des composés utilisés dans la présente invention données ci-dessous sont valables pour l'ensemble des objets de l'invention.

Au sens de la présente invention on entend par phosphate d'amidon les produits issus de l'estérification d'un amidon par un dérivé phosphoré conduisant à la formation d'au moins une liaison ester entre le dérivé phosphoré et au moins un groupement hydroxyle de l'amidon.

Les amidons utilisables dans la présente invention sont plus particulièrement des macromolécules sous forme de polymères constitués de motifs élémentaires qui sont des unités anhydroglucose. Le nombre de ces motifs et leur assemblage permettent de distinguer l'amylose (polymère linéaire) et l'amylopectine (polymère ramifié). Les proportions relatives d'amylose et d'amylopectine, ainsi que leur degré de polymérisation, varient en fonction de l'origine botanique des amidons.

Les molécules d'amidons utilisés dans la présente invention peuvent avoir comme origine botanique les céréales ou encore les tubercules. Ainsi, les amidons sont par exemple choisis parmi les amidons de maïs, de riz, de manioc, de tapioca, d'orge, de pomme de terre, de blé, de sorgho, de pois.

Les amidons se présentent généralement sous la forme d'une poudre blanche, insoluble dans l'eau froide, dont la taille des particules élémentaires va de 3 à 100 microns.

Selon l'invention, les amidons peuvent être éventuellement hydroxyalkylés en C1-C6 ou acylés en C1-C6 (de préférence acétylé). Les amidons peuvent également avoir subi des traitements thermiques.

On peut obtenir soit des phosphates de monoamidon (du type Am-O-PO-(OX)₂), des phosphates de diamidon (du type Am-O-PO-(OX)-O-Am) ou même de triamidon (du type Am-O-PO- (O-Am)₂) ou leurs mélanges.

X désigne notamment les métaux alcalins (par exemple sodium ou potassium), les métaux alcalinoterreux (par exemple calcium, magnésium), les sels d'ammoniaque, les sels d'amines comme ceux de la monoéthanolamine, la diéthanolamine, la triéthanolamine, l'amino-3 propanediol-1,2, les sels ammoniums issus des aminoacides basiques tels que la lysine, l'arginine, la sarcosine, l'ornithine, la citrulline.

Les composés phosphorés peuvent être par exemple du tripolyphosphate de sodium, de l'orthophosphate de sodium, de l'oxychlorure de phosphore ou du trimétaphosphate de sodium.

On utilisera préférentiellement des phosphates de diamidon ou des composés riches en phosphate de diamidon comme le produit proposé sous les références PREJEL VA-70-T AGGL (phosphate de diamidon de manioc hydroxypropylé gélatinisé) ou PREJEL TK1 (phosphate de diamidon de manioc gélatinisé) ou PREJEL 200 (phosphate de diamidon de manioc acétylé gélatinisé) par la Société AVEBE.

Le ou les phosphates d'amidon sont utilisés de préférence en une quantité allant de 0,01 à 20% en poids par rapport au poids total de la composition. Plus préférentiellement, cette quantité va de 0,05 à 15% en poids par rapport au poids total de la composition et encore plus préférentiellement allant de 0,1 à 10% en poids.

De manière encore plus générale, au sens de la présente invention, l'expression "polymère cationique" désigne tout polymère contenant des groupements cationiques et/ou des groupements ionisables en groupements cationiques.

L'expression "non siliconé" signifie que le polymère cationique ne contient pas de silicium.

Les polymères cationiques utilisés ont généralement une masse moléculaire moyenne en nombre allant de 500 à 5.10⁶ environ, et de préférence allant de 10³ à 3.10⁶ environ.

Les polymères cationiques sont choisis parmi
(2) Les celluloses cationiques. Parmi les celluloses cationiques, on peut citer plus particulièrement les dérivés d'éthers de cellulose comportant des groupements ammonium quaternaires, les copolymères de cellulose cationiques ou les dérivés de cellulose greffés avec un monomère hydrosoluble d'ammonium.

Les dérivés d'éthers de cellulose comportant des groupements ammonium quaternaires décrits dans le brevet français 1 492 597, et en particulier les polymères commercialisés sous les dénominations "JR" (JR 400, JR 125, JR 30M) ou "LR" (LR 400, LR 30M) par la Société UNION CARBIDE Corporation. Ces polymères sont également définis dans le dictionnaire CTFA comme des ammonium quaternaires d'hydroxyéthylcellulose ayant réagi avec un époxyde substitué par un groupement triméthylammonium.

Les copolymères de cellulose cationiques ou les dérivés de cellulose greffés avec un monomère hydrosoluble d'ammonium quaternaire, sont décrits notamment dans le brevet US 4 131 576, tels que les hydroxyalkyl celluloses, comme les hydroxyméthyl-, hydroxyéthyl- ou hydroxypropyl celluloses greffées notamment avec un sel de méthacryloyléthyl triméthylammonium, de méthacrylmidopropyl triméthylammonium, de diméthyl-diallylammonium.

Les produits commercialisés répondant à cette définition sont plus particulièrement les produits vendus sous la dénomination "CELQUAT L 200" et "CELQUAT H 100" par la Société NATIONAL STARCH.
(5) les dérivés de polyaminoamides résultant de la condensation de polyalcoylènes polyamines avec des acides polycarboxyliques suivie d'une alcoylation par des agents bifonctionnels. On peut citer par exemple les polymères acide adipique-diacoylaminohydroxyalcoyldialoylène triamine dans lesquels le radical alcoyle comporte de 1 à 4 atomes de carbone et désigne de préférence méthyle, éthyle, propyle. De tels polymères sont notamment décrits dans le brevet français 1.583.363.

Parmi ces dérivés, on peut citer plus particulièrement les polymères acide adipique/diméthylaminohydroxypropyl/diéthylène triamine vendus sous la dénomination "Cartaretine F, F4 ou F8" par la société Sandoz.
(7) les cyclopolymères d'alkyl diallyl amine ou de dialkyl diallyl ammonium tels que les homopolymères ou copolymères comportant comme constituant principal de la chaîne des motifs répondant aux formules (VI) ou (VI') : 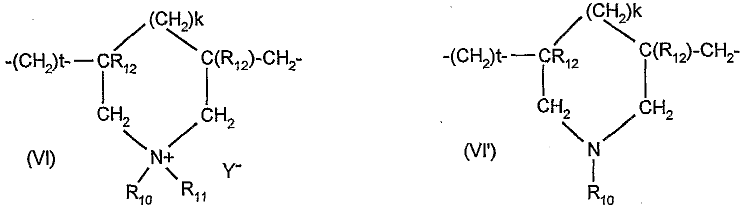 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate,
à l'exception du copolymère de chlorure de diallyldiméthylammonium et d'acrylamide notamment commercialisés sous la dénomination "MERQUAT 550".

Ces polymères sont notamment décrits dans le brevet français 2.080.759 et dans son certificat d'addition 2.190.406.

R₁₀ et R₁₁, indépendamment l'un de l'autre, désignent de préférence un groupement alkyle ayant de 1 à 4 atomes de carbone.

Parmi les polymères définis ci-dessus, on peut citer plus particulièrement les homopolymères comportant comme constituant principal de la chaîne des motifs répondant aux formules (VI) ou (VI') tels que notamment l'homopolymère de chlorure de diméthyldiallylammonium par exemple vendu sous la dénomination "MERQUAT 100" par la société CALGON (et ses homologues de faibles masses moléculaires moyenne en poids).
(8) les polymères de diammonium quaternaire comprenant des motifs récurrents répondant à la formule : 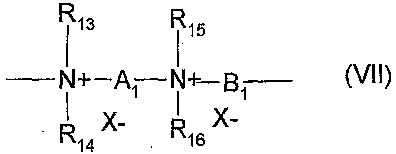 formule (VII) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles contenant éventuellement un second hétéroatome autre que l'azote ou bien R₁₃, R₁₄, R₁₅ et R₁₆ représentent un radical alkyle en C1-C6 linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
   dans lequel D désigne :
   a) un reste de glycol de formule : -O-Z-O-, où Z désigne un radical hydrocarboné linéaire ou ramifié ou un groupement répondant à l'une des formules suivantes :

      -(CH2-CH2-O)x-CH2-CH2-

      -[CH2-CH(CH3)-O]y-CH2-CH(CH3)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH2-CH2-S-S-CH2-CH2- ;
   d) un groupement uréylène de formule : -NH-CO-NH- ;

De préférence, X⁻ est un anion tel que le chlorure ou le bromure.

Ces polymères ont une masse moléculaire moyenne en nombre généralement allant de 1000 à 100000.

Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement les polymères qui sont constitués de motifs récurrents répondant à la formule : 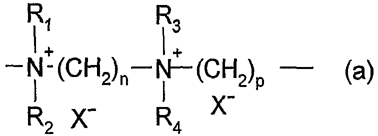 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).
(10) Les polymères quaternaires de vinylpyrrolidone et de vinylimidazole tels que par exemple les produits commercialisés sous les dénominations LUVIQUAT® FC 905, FC 550 et FC 370 par la société B.A.S.F.
(12) Les polymères (homopolymère ou copolymère) réticulés ou non réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide ou de méthacrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de « SALCARE^{®} SC 92 » par la Société ALLIED COLLOIDS. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société ALLIED COLLOIDS.

Parmi tous les polymères cationiques susceptibles d'être utilisés dans le cadre de la présente invention, on préfère mettre en oeuvre les dérivés d'éther de cellulose quaternaires tels que les produits vendus sous la dénomination « JR 400 » par la Société UNION CARBIDE CORPORATION, les homopolymères de chlorure de diméthyldiallylammonium, vendus sous la dénomination « MERQUAT 100 »,» par la société CALGON, les polymères quaternaires de vinylpyrrolidone et de vinylimidazole, les homopolymères ou copolymères réticulés ou non réticulés de sels de méthacryloyloxyalkyl(C₁-C₄) trialkyl(C₁-C₄)ammonium et leurs mélanges.

Selon l'invention, le ou les polymères cationiques peuvent représenter de 0,001 % à 20 % en poids, de préférence de 0,01 % à 10% en poids et plus particulièrement de 0,1 à 5% en poids par rapport au poids total de la composition finale.

Selon un mode de réalisation particulièrement préféré, les compositions selon l'invention comprennent en outre au moins une silicone ou un autre agent bénéfique pour les cheveux tels que notamment les esters d'acides carboxyliques en C1-C30 et de d'alcools mono ou polyhydroxylés enC1-C30, les huiles végétales, animales, minérales ou de synthèse, les cires, les céramides, les pseudocéramides.

Les silicones utilisables conformément à l'invention sont en particulier des polyorganosiloxanes insolubles dans la composition et peuvent se présenter sous forme d'huiles, de cires, de résines ou de gommes.

Les organopolysiloxanes sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones sont plus particulièrement choisies parmi celles possédant un point d'ébullition allant de 60° C à 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHONE POULENC, le décaméthylcyclopentasiloxane commercialisé sous le nom de "VOLATILE SILICONE 7158" par UNION CARBIDE, "SILBIONE 70045 V 5" par RHONE POULENC, ainsi que leurs mélanges.
   On peut également citer les cyclocopolymères du type diméthylsiloxanes/ méthylakylsiloxane, tel que la "SILICONE VOLATILE FZ 3109" commercialisée par la société UNION CARBIDE, de structure chimique : avec D : 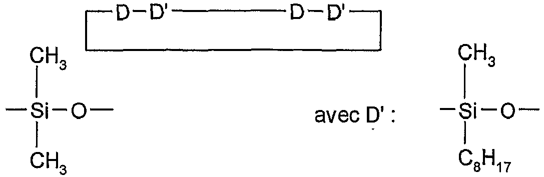
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

On utilise de préférence des silicones non volatiles et plus particulièrement des polyalkylsiloxanes, des polyarylsiloxanes, des polyalkylarylsiloxanes, des gommes et des résines de silicones, des polyorganosiloxanes modifiés par des groupements organofonctionnels ainsi que leurs mélanges.

Ces silicones sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les polyalkylarylsiloxanes sont particulièrement choisis parmi les polydiméthyl méthylphénylsiloxanes, les polydiméthyl diphénylsiloxanes linéaires et/ou ramifiés de viscosité de 1.10⁻⁵ à 5.10⁻²m²/s à 25°C.

Parmi ces polyalkylarylsiloxanes on peut citer à titre d'exemple les produits commercialisés sous les dénominations suivantes :
- les huiles SILBIONE de la série 70 641 de RHONE POULENC ;
- les huiles des séries RHODORSIL 70 633 et 763 de RHONE POULENC ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Les gommes de silicone utilisables conformément à l'invention sont notamment des polydiorganosiloxanes ayant des masses moléculaires moyennes en nombre élevées allant de 200 000 à 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- polydiméthylsiloxane
- les gommes polydiméthylsiloxanes/méthylvinylsiloxane,
- polydiméthylsiloxane/diphénylsiloxane,
- polydiméthylsiloxane/phénylméthylsiloxane,
- polydiméthylsiloxaneldiphénylsiloxanelméthylvinyisiloxane.

Des produits plus particulièrement utilisables conformément à l'invention sont des mélanges tels que :
- les mélanges formés à partir d'un polydiméthylsiloxane hydroxylé en bout dé chaîne (dénommé diméthiconol selon la nomenclature du dictionnaire CTFA) et d'un poly-diméthylsiloxane cyclique (dénommé cyclométhicone selon la nomenclature du dictionnaire CTFA) tel que le produit Q2 1401 commercialisé par la société DOW CORNING ;
- les mélanges formés à partir d'une gomme polydiméthylsiloxane avec une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle.
Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organo modifiées utilisables conformément à l'invention sont des silicones telles que définies précédemment et comportant dans leur structure un ou plusieurs groupements organofonctionnels fixés par l'intermédiaire d'un radical hydrocarboné.

Parmi les silicones organomodifiées, on peut citer les polyorganosiloxanes comportant :
- des groupements polyéthylèneoxy et/ou polypropylèneoxy comportant éventuellement des groupements alkyle en C₆-C₂₄ tels que les produits dénommés diméthicone copolyol commercialisé par la société DOW CORNING sous la dénomination DC 1248 ou les huiles SILWET L 722, L 7500, L 77, L 711 de la société UNION CARBIDE et l'alkyl (C₁₂) méthicone copolyol commercialisée par la société DOW CORNING sous la dénomination Q2 5200 ;
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements thiols comme les produits commercialisés sous les dénominations "GP 72 A" et "GP 71" de GENESEE ;
- des groupements alcoxylés comme le produit commercialisé sous la dénomination "SILICONE COPOLYMER F-755" par SWS SILICONES et ABIL WAX 2428, 2434 et 2440 par la société GOLDSCHMIDT ;
- des groupements hydroxylés comme les polyorganosiloxanes à fonction hydroxyalkyle décrits dans la demande de brevet français FR-A-85 16334 répondant à la formule (IX) : 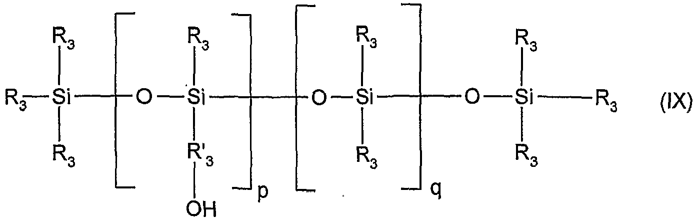 dans laquelle les radicaux R₃ identiques ou différents sont choisis parmi les radicaux méthyle et phényle ; au moins 60 % en mole des radicaux R₃ désignant méthyle ; le radical R'₃ est un chaînon alkylène divalent hydrocarboné en C₂-C₁₈ ; p va de 1 à 30 inclus ; q va de 1 à 150 inclus ;
- des groupements acyloxyalkyle tels que par exemple les polyorganosiloxanes décrits dans le brevet US-A-4957732 et répondant à la formule (X) : 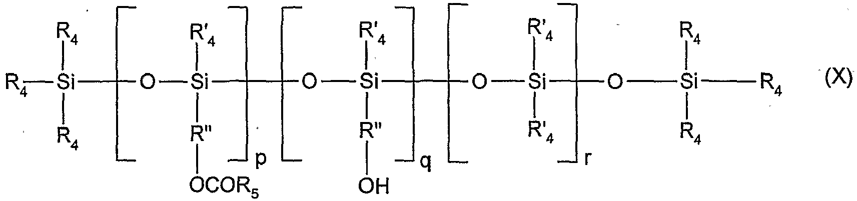 dans laquelle :
   R₄ désigne un groupement méthyle, phényle, -OCOR₅, hydroxyle, un seul des radicaux R₄ par atome de silicium pouvant être OH ;
   R'₄ désigne méthyle, phényle ; au moins 60 % en proportion molaire de l'ensemble des radicaux R₄ et R'₄ désignant méthyle ;
   R₅ désigne alkyle ou alcényle en C₈-C₂₀ ;
   R" désigne un radical alkylène hydrocarboné divalent, linéaire ou ramifié en C₂-C₁₈ ;
   r va de 1 à 120 inclus ;
   p va de 1 à 30 ;
   q est égal à 0 ou est inférieur à 0,5 p, p + q allant de 1 à 30 ; les polyorganosiloxanes de formule (VI) peuvent contenir des groupements : 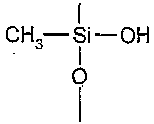 dans des proportions ne dépassant pas 15 % de la somme p + q + r.
- des groupements anioniques du type carboxylique comme par exemple dans les produits décrits dans le brevet EP 186 507 de la société CHISSO CORPORATION, ou de type alkylcarboxyliques comme ceux présents dans le produit X-22-3701E de la société SHIN-ETSU ; 2-hydroxyalkylsulfonate ; 2-hydroxyalkylthiosulfate tels que les produits commercialisés par la société GOLDSCHMIDT sous les dénominations "ABIL S201" et "ABIL S255".
- des groupements hydroxyacylamino, comme les polyorganosiloxanes décrits dans la demande EP 342 834. On peut citer par exemple le produit Q2-8413 de la société DOW CORNING.

Selon l'invention, on peut également utiliser des silicones comprenant une portion polysiloxane et une portion constituée d'une chaîne organique non-siliconée, l'une des deux portions constituant la chaîne principale du polymère l'autre étant greffée sur la dite chaîne principale. Ces polymères sont par exemple décrits dans les demandes de brevet EP-A-412 704, EP-A-412 707, EP-A-640 105 et WO 95/00578, EP-A-582 152 et WO 93/23009 et les brevets US 4,693,935, US 4,728,571 et US 4,972,037. Ces polymères sont de préférence anioniques ou non ioniques.

De tels polymères sont par exemple les copolymères susceptibles d'être obtenus par polymérisation radicalaire à partir du mélange de monomères constitué par :
a) 50 à 90% en poids d'acrylate de tertiobutyle ;
b) 0 à 40% en poids d'acide acrylique ;
c) 5 à 40% en poids de macromère siliconé de formule : 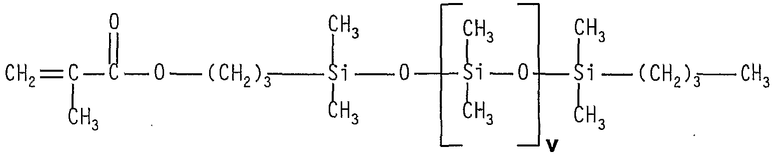
avec v étant un nombre allant de 5 à 700 ; les pourcentages en poids étant calculés par rapport au poids total des monomères.

D'autres exemples de polymères siliconés greffés sont notamment des polydiméthylsiloxanes (PDMS) sur lesquels sont greffés, par l'intermédiaire d'un chaînon de raccordement de type thiopropylène, des motifs polymères mixtes du type acide poly(méth)acrylique et du type poly(méth)acrylate d'alkyle et des polydiméthylsiloxanes (PDMS) sur lesquels sont greffés, par l'intermédiaire d'un chaînon de raccordement de type thiopropylène, des motifs polymères du type poly(méth)acrylate d'isobutyle.

Selon l'invention, on peut également utiliser des polyuréthanes siliconés, notamment ceux décrits dans les demandes de brevet EP 0 751 162, EP 0 619 111.

Selon l'invention, toutes les silicones peuvent également être utilisées sous forme d'émulsions, de nanoémulsions ou de micrémulsions.

Les polyorganosiloxanes particulièrement préférés conformément à l'invention sont :
- les silicones non volatiles choisies dans la famille des polyalkylsiloxanes à groupements terminaux triméthylsilyle telles que les huiles ayant une viscosité allant de 0,2 à 2,5 m²/s à 25° C telles que les huiles de la séries DC200 de DOW CORNING en particulier celle de viscosité 60 000 Cst, des séries SILBIONE 70047 et 47 et plus particulièrement l'huile 70 047 V 500 000 commercialisées par la société RHONE POULENC, les polyalkylsiloxanes à groupements terminaux diméthylsilanol tels que les diméthiconol ou les polyalkylarylsiloxanes tels que l'huile SILBIONE 70641 V 200 commercialisée par la société RHONE POULENC ;
- la résine d'organopolysiloxane commercialisée sous la dénomination DOW CORNING 593 ;
- les polysiloxanes à groupements aminés tels que les amodiméthicones ou les triméthylsilylamodiméthicone ;

Selon l'invention, les esters d'acides carboxyliques en C1-C30 et de d'alcools mono ou polyhydroxylés en C1-C30 sont choisis notamment parmi le myristate d'isopropyle, l'isononanoate d'isononyle, et leurs mélanges.

Selon l'invention, les huiles végétales sont choisies notamment parmi l'huile d'avocat, l'huile d'olive et leurs mélanges.

Selon l'invention, les silicones additionnelles ou les autres agents bénéfiques additionnels peuvent représenter de 0,001 % à 20 % en poids, de préférence de 0,01 % à 10% en poids et plus particulièrement de 0,1 à 5% en poids par rapport au poids total de la composition finale.

Les compositions de l'invention contiennent en outre au moins un agent tensioactif qui est généralement présent en une quantité comprise allant de 0,01% à 50% en poids environ, de préférence allant de 0,1% à 40% et encore plus préférentiellement allant de 0,5% à 30%, par rapport au poids total de la composition.

Cet agent tensioactif est choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Leur nature ne revêt pas, dans le cadre de la présente invention, de caractère véritablement critique.

Ainsi, à titre d'exemple de tensioactifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylàrylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryl désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet, égard "Handbook of Surfactants" par M.R. PORTER, éditions Blackie & Son (Glasgow and London), 1991, pp 116-178) et leur nature ne revêt pas, dans le cadre de la présente invention, de caractère critique. Ainsi, ils peuvent être notamment choisis parmi (liste non limitative) les alcools, les alpha-diols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les aminés grasses polyéthoxylées ayant de préférence 2 à 30 moles d'oxyde d'éthylène ; les esters d'acides gras du sorbitan oxyéthylénés ayant de 2 à 30 moles d'oxyde d'éthylène.; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) aminés ou les oxydes de N-acylaminopropylmorpholine. On notera que les alkylpolyglycosides constituent des tensioactifs non-ioniques rentrant particulièrement bien dans le cadre de la présente invention.

### (iii) Tensioactif(s) amphotère(s):

Les agents tensioactifs amphotères, dont la nature ne revêt pas dans le cadre de la présente invention de caractère critique, peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et contenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.

Parmi les dérivés d'amines, on peut citer les produits commercialisés sous les dénomination MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ;
et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroampho-diacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.

A titre d'exemple on peut citer le cocoamphodiacetate commercialisé sous la dénomination commerciale MIRANOL C2M concentré par la société RHONE POULENC.
(iv) Les tensioactifs cationiques peuvent être, choisis parmi :
   A) les sels d'ammonium quaternaires de la formule générale (IV) suivante : 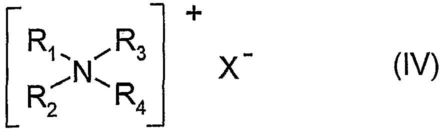 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
      , et
      a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
         R4 désigne un radical alkyle, linéaire ou ramifié, comportant de 16 à 30 atomes de carbone.
         De préférence le tensioactif cationique est un sel (par exemple chlorure) de béhényl triméthyl ammonium.
      b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
         R3 et R4, identiques ou différents, désignent un radical alkyle, linéaire ou ramifié, comportant de 12 à 30 atomes de carbone, ledit radical comprenant au moins une fonction ester ou amide.
         R3 et R4 sont notamment choisis parmi les radicaux alkyl(C₁₂-C₂₂)amido alkyle(C₂-C₆), alkyl(C₁₂-C₂₂)acétate ;
         De préférence le tensioactif cationique est un sel (par exemple chlorure) de stéaramidopropyl diméthyl (myristylacétate) ammonium.
   B) - les sels d'ammonium quaternaire de l'imidazolinium, comme par exemple celui de formule (V) suivante : 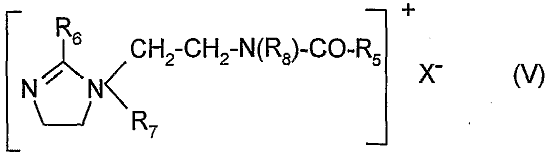 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
   C) - les sels de diammonium quaternaire de formule (VI) : 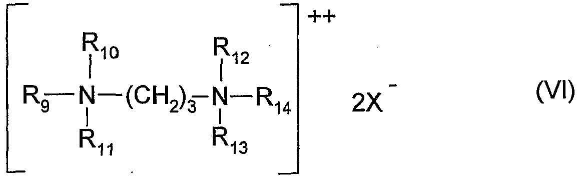 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
   D) - les sels d'ammonium quaternaire contenant au moins une fonction ester de formule (VII) suivante : 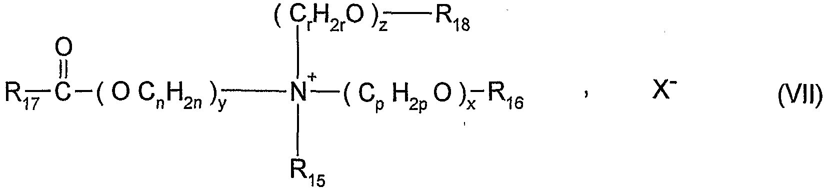 dans laquelle :
      - R₁₅ est choisi parmi les radicaux alkyles en C₁-C₆ et les radicaux hydroxyalkyles ou dihydroxyalkyles en C₁-C₆ ;
      - R₁₆ est choisi parmi :
         - le radical 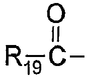
         - les radicaux R₂₀ hydrocarbonés en C₁-C₂₂ linéaires ou ramifiés, saturés ou insaturés,
         - l'atome d'hydrogène,
      - R₁₈ est choisi parmi :
         - le radical 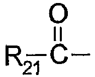
         - les radicaux R₂₂ hydrocarbonés en C₁-C₆ linéaires ou ramifiés, saturés ou insaturés,
         - l'atome d'hydrogène,
      - R₁₇, R₁₉ et R₂₁, identiques ou différents, sont choisis parmi les radicaux hydrocarbonés en C₇-C₂₁, linéaires ou ramifiés, saturés ou insaturés ;
      - n, p et r, identiques ou différents, sont des entiers valant de 2 à 6 ;
      - y est un entier valant de 1 à 10 ;
      - x et z, identiques ou différents, sont des entiers valant de 0 à 10 ;
      - X- est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (VII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 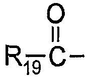
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés ;
- R₁₈ est choisi parmi :
   - le radical 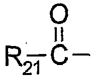
   - l'atome d'hydrogène ;

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

Parmi les sels d'ammonium quaternaire on préfère les sels (par exemple chlorure) de béhényltriméthylammonium, ou encore, les sels (par exemple chlorure) de stéaramidopropyldiméthyl (myristyl acétate) ammonium commercialisé sous la dénomination «CERAPHYL 70» par la société VAN DYK, les sels (par exemple chlorure) de palmitylamidopropyl triméthyl ammonium, le Quaternium-27 ou le Quaternium-83 commercialisés par la société WITCO.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange constitué d'au moins un agent tensioactif anionique et d'au moins un agent tensioactif amphotère.

La composition de l'invention peut également contenir au moins un additif choisi parmi les épaississants, les parfums, les agents nacrants, les conservateurs, les filtres solaires siliconés ou non, les vitamines, les provitamines, les polymères anioniques ou non ioniques, les protéines non cationiques, les hydrolysats de protéines non cationiques, l'acide méthyl-18 eicosanoique, les hydroxyacides, les vitamines, les provitamines telles que le panthénol, et tout autre additif classiquement utilisé dans le domaine cosmétique qui n'affecte pas les propriétés des compositions selon l'invention.

Ces additifs sont éventuellement présents dans la composition selon l'invention dans des proportions pouvant aller de 0,001 à 20% en poids par rapport au poids total de la composition. La quantité précise de chaque additif est déterminée facilement par l'homme du métier selon sa nature et sa fonction.

Selon un mode de réalisation particulièrement préféré, les compositions selon l'invention comprennent un phosphate d'amidon, au moins un polymère cationique tel que défini ci-dessus, au moins un tensioactif cationique et au moins une silicone.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse.

Le ou les tensioactifs constituant la base lavante peuvent être indifféremment choisis, seuls ou en mélanges, au sein des tensioactifs anioniques, amphotères, non ioniques et cationiques tels que définis ci-dessus. La base lavante contient au moins un tensioactif détergent.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHONE POULENC sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes en particulier la cocobétaïne commercialisée sous la dénomination "DEHYTON AB 30" en solution aqueuse à 32 % de MA par la société HENKEL.

La quantité et la qualité des tensioactifs de la base lavante sont celles suffisantes pour conférer à la composition finale un pouvoir moussant et/ou détergent satisfaisant.

Ainsi, selon l'invention, les tensioactifs peuvent représenter de 4 % à 50 % en poids, de préférence de 6 % à 35 % en poids, et encore plus préférentiellement de 8 % à 25 % en poids, du poids total de la composition finale.

L'invention a encore pour objet un procédé de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle contient avantageusement au moins un tensioactif cationique, sa concentration généralement allant de 0,1 à 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.

Dans les exemples, MA signifie matière active.

### EXEMPLE 1

On a réalisé un après-shampooing conforme à l'invention de composition suivante :
- Phosphate de diamidon (PREJEL VA-70-T de AGGL) 2,5 g MA
- Chlorure de béhényl triméthyl ammonium 1,8 g MA
- Homopolymère de chlorure de méthacrylate d'éthyl triméthyl ammonium (SYNTHALEN CR de 3V) 1,5 g MA
- Amodimethicone (BELSIL ADM 6057 E de WACKER) 1,7 gMA
- Eau qsp 100 g

La composition a une texture gélifiée et très fondante lors de l'application sur des cheveux humides. Sa rinçabilité est bonne. Les cheveux mouillés ne sont pas chargés et la mise en forme est aisée.

### EXEMPLE 2

On a réalisé un après-shampooing conforme à l'invention de composition suivante :
- Phosphate de diamidon (PREJEL VA-70-T de AGGL) 5 g MA
- Homopolymère de chlorure de méthacrylate d'éthyl triméthyl ammonium réticulé (SALCARE SC 95 de CIBA) 1 g MA
- Divinyldimethicone /diméthicone réticulé en émulsion cationique à 65% de MA (DC2-1997 de DOW CORNING) 5 gMA
- Eau qsp 100 g

Les cheveux traités ont les mêmes propriétés que ceux traités avec la composition de l'exemple 1.

### EXEMPLE 3

On a réalisé un après-shampooing conforme à l'invention de composition suivante :
- Phosphate de diamidon (PREJEL VA-70-T de AGGL) 8 g MA
- Chlorure de palmitylamidopropyl triméthyl ammonium à 60% dans le propylèneglycol (VARISOFT PATC de GOLDSCHMIDT) 4 g MA
- Homopolymère de chlorure de méthacrylate d'éthyl triméthyl ammonium réticulé (SALCARE SC 95 de CIBA) 0,1 g MA
- Myristate d'isopropyle 2 g
- Eau qsp 100 g

Les cheveux traités ont les mêmes propriétés que ceux traités avec la composition de l'exemple 1.

### EXEMPLE 4

On a réalisé un après-shampooing conforme à l'invention de composition suivante :
- Phosphate de diamidon (PREJEL VA-70-T de AGGL) 6 g MA
- Chlorure de béhényl triméthyl ammonium 2,5 g MA
- Copolymère de méthacrylamide et de chlorure de méthacrylate d'éthyl triméthyl ammonium (Polyquaternium-15) (ROHAGIT KF 720 de ROHM) 0,8 g MA
- Divinyldimethicone /diméthicone réticulé en émulsion cationique (DC2-1997 de DOW CORNING) 1,5 gMA
- Eau qsp 100 g

Les cheveux traités ont les mêmes propriétés que ceux traités avec la composition de l'exemple 1.

### EXEMPLE 5

On a réalisé un après-shampooing conforme à l'invention de composition suivante :
- Phosphate de diamidon (PREJEL VA-70-T de AGGL) 7 g MA
- Lauryléthersulfate de sodium à 2,2 moles d'oxyde d'éthylène 1 g MA
- Polyquaternium-10 (JR 400 de AMERCHOL) 1,2 g MA
- Huile d'avocat 3 g
- Eau qsp 100 g

Les cheveux traités ont les mêmes propriétés que ceux traités avec la composition de l'exemple 1.

## Revendications

1. Composition cosmétique, **caractérisée par le fait qu'**elle comprend, dans un milieu cosmétiquement acceptable, :
- au moins un agent tensioactif choisi parmi les tensioactifs anioniques, non ioniques, amphotères, cationiques et leurs mélanges;
- au moins un phosphate d'amidon et
- au moins un polymère cationique non siliconé choisi parmi :
(2) les celluloses cationiques,
(5) les dérivés de polyaminoamides résultant de la condensation de polyalcoylènes polyamines avec des acides polycarboxyliques suivie d'une alcoylation par des agents bifonctionnels,
(7) les cyclopolymères d'alkyl diallyl amine ou de dialkyl diallyl ammonium, à l'exception du copolymère de chlorure de diallyldiméthylammonium et d'acrylamide,
(8) les polymères de diammonium quaternaire comprenant des motifs récurrents répondant à la formule : 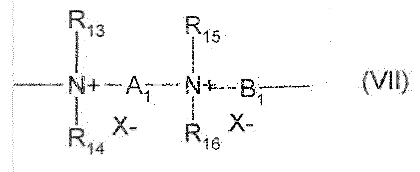 dans laquelle :
R13, R14, R15 et R16, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R13, R14, R15 et R16, ensemble ou séparément, constituent avec les atomes d'azote auxquels ils sont rattachés des hétérocycles contenant éventuellement un second hétéroatome autre que l'azote ou bien R13, R14, R15 et R16 représentent un radical alkyle en C1-C6 linéaire ou ramifié substitué par un groupement nitrile, ester, acyle, amide ou -CO-O-R17-D ou -CO-NH-R17-D où R17 est un alkylène et D un groupement ammonium quaternaire ;
A1 et B1 représentent des groupements polyméthyléniques contenant de 2 à 20 atomes de carbone pouvant être linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ désigne un anion dérivé d'un acide minéral ou organique;
A1, R13 et R15 peuvent former avec les deux atomes d'azote auxquels ils sont rattachés un cycle pipérazinique ; en outre si A1 désigne un radical alkylène ou hydroxyalkylène linéaire ou ramifié, saturé ou insaturé, B1 peut également désigner un groupement (CH2)n-CO-D-OC-(CH2)n-
dans lequel D désigne :
a) un reste de glycol de formule : -O-Z-O-, où Z désigne un radical hydrocarboné linéaire ou ramifié ou un groupement répondant à l'une des formules suivantes :
-(CH2-CH2-O)x-CH2-CH2-
-[CH2-CH(CH3)-O]y-CH2-CH(CH3)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH2-CH2-S-S-CH2-CH2- ;
d) un groupement uréylène de formule : -NH-CO-NH- ;
(10) les polymères quaternaires de vinylpyrrolidone et de vinylimidazole,
(12) les polymères réticulés ou non réticulés de sels de méthacryloyloxyalkyl(C₁-C4)trialkyl(C1-C4)ammonium.

2. Composition selon la revendication 1, **caractérisée par le fait que** le phosphate d'amidon est un ester de composés phosphorés et d'au moins un amidon.

3. Composition selon la revendication précédente, **caractérisée en ce que** le composé phosphoré est choisi parmi le tripolyphosphate de sodium, l'orthophosphate de sodium, l'oxychlorure de phosphore et le trimétaphosphate de sodium.

4. Composition selon l'une quelconque des revendications 1 à 3, **caractérisée par le fait que** les amidons sont choisis parmi les amidons de maïs, de riz, de manioc, de tapioca, d'orge, de pomme de terre, de blé, de sorgho, de pois.

5. Composition selon l'une quelconque des revendications 1 à 4, **caractérisée par le fait que** l'amidon est hydroxyalkylé en C1-C6 ou acylé en C1-C6.

6. Composition selon l'une quelconque des revendications 1 à 5, **caractérisée par le fait que** le phosphate d'amidon est choisi parmi les phosphates de mono amidon, les phosphates de diamidon, les phosphates de triamidon et leurs mélanges.

7. Composition selon la revendication 6, **caractérisée par le fait que** le phosphate d'amidon est choisi parmi les phosphates de diamidon.

8. Composition selon l'une quelconque des revendications 1 à 5, **caractérisée par le fait que** le phosphate d'amidon est choisi parmi les phosphates de diamidon de manioc hydroxypropylés gélatinisés, les phosphates de diamidon de manioc acétylés gélatinisés, les phosphates de diamidon de manioc gélatinisés.

9. Composition selon l'une quelconque des revendications précédentes, **caractérisée par le fait que** le polymère cationique non siliconé est choisi parmi :
(2) les dérivés d'éthers de cellulose comportant des groupements ammonium quaternaires, les copolymères de cellulose cationiques ou les dérivés de celluloses greffés avec un monomère hydrosoluble d'ammonium;
(5) les polymères acide adipique-diacoylaminohydroxyalcoyldialoylène triamine dans lesquels le radical alcoyle comporte de 1 à 4 atomes de carbone;
(7) les homopolymères ou copolymères comportant comme constituant principal de la chaîne des motifs répondant aux formules (VI) ou (VI') : 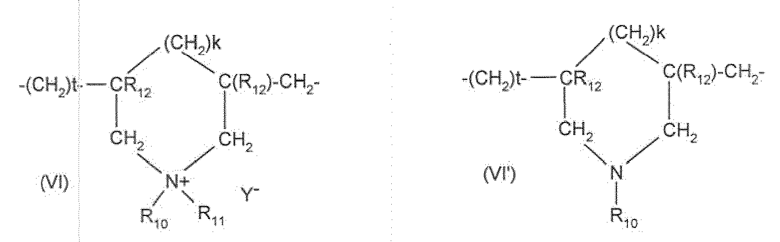 dans lesquelles k et t sont égaux à 0 ou 1, la somme k + t étant égale à 1; R12 désigne un atome d'hydrogène ou un radical méthyle; R10 et R11, indépendamment l'un de l'autre, désignent un groupement alkyle ayant de 1 à 22 atomes de carbone, un groupement hydroxyalkyle dans lequel le groupement alkyle a de préférence 1 à 5 atomes de carbone, un groupement amidoalkyle inférieur (C1-C4) ou R10 et R11 peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate,
à l'exception du copolymère de chlorure de diallyldiméthylammonium et d'acrylamide;
(8) les polymères de diammonium quaternaire constitués de motifs récurrents répondant à la formule : 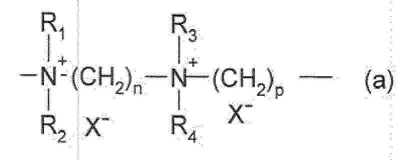 dans laquelle R1, R2, R3 et R4, identiques ou différents, désignent un radical alkyle ou hydroxyalkyle ayant de 1 à 4 atomes de carbone environ, n et p sont des nombres entiers variant de 2 à 20 environ et X- est un anion dérivé d'un acide minéral ou organique;
(12) les polymères obtenus par homopolymérisation du diméthylaminoéthylméthacrylate quaternisé par le chlorure de méthyle, ou par copolymérisation de l'acrylamide ou de méthacrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide.

10. Composition selon l'une des revendications précédentes, **caractérisée par le fait que** le polymère cationique non siliconé est choisi parmi les homopolymères du chlorure de diallyldiméthylammonium.

11. Composition selon l'une des revendications 1 à 9 , **caractérisée par le fait que** les celluloses cationiques sont choisies parmi les hydroxyéthylcelluloses ayant réagi avec un époxyde substitué par un groupement triméthylammonium.

12. Composition selon l'une quelconque des revendications 1 à 11, **caractérisée par le fait qu'**elle comprend en outre au moins une silicone.

13. Composition selon la revendication 12, **caractérisée par le fait que** les silicones sont choisies parmi les polyorganosiloxanes insolubles dans la composition.

14. Composition selon l'une quelconque des revendications 12 ou 13, **caractérisée par le fait que** les silicones sont des polyorganosiloxanes non volatils choisis parmi les polyalkylsiloxanes, les polyarylsiloxanes, les polyalkylarylsiloxanes, les gommes et résines de silicones, les polyorganosiloxanes modifiés par des groupements organofonctionnels ainsi que leurs mélanges.

15. Composition selon la revendication 14, **caractérisée par le fait que** :
(a) les polyalkylsiloxanes sont choisis parmi :
- les polydiméthylsiloxanes à groupements terminaux triméthylsilyle ;
- les polydiméthylsiloxanes à groupements terminaux diméthylsilanol ;
- les polyalkyl(C₁-C₂₀)siloxanes ;
(b) les polyalkylarylsiloxanes sont choisis parmi :
- les polydiméthylméthylphénylsiloxanes, les polydiméthyldiphényl siloxanes linéaires et/ou ramifiés de viscosité allant de 1.10⁻⁵ et 5.10⁻²m²/s à 25°C ;
(c) les gommes de silicone sont choisies parmi les polydiorganosiloxanes ayant des masses moléculaires moyennes en nombre allant de 200 000 et 1 000 000 utilisés seuls ou sous forme de mélange dans un solvant ;
(d) les résines sont choisies parmi les résines constituées d'unités : R₃ Si O_{1/2}, R₂ Si O_{2/2}, R Si O_{3/2}, Si O_{4/2}
dans lesquelles R représente un groupement hydrocarboné ayant de 1 à 16 atomes de carbone ou un groupement phényle ;
(e) les silicones organo modifiées sont choisies parmi les silicones comportant dans leur structure un ou plusieurs groupements organofonctionnels fixés par l'intermédiaire d'un radical hydrocarboné.

16. Composition selon l'une quelconque des revendications 12 à 15, **caractérisée par le fait que** les silicones sont choisies parmi les polyalkylsiloxanes à groupements terminaux triméthylsilyle, les polyalkylsiloxanes à groupements germinaux diméthylsilanol, les polyalkylarylsiloxanes, les mélanges de deux PDMS constitués d'une gomme et d'une huile de viscosités différentes, les mélanges d'organosiloxanes et de silicones cycliques, les résines d'organopolysiloxanes, les polysiloxanes à groupements aminés.

17. Composition selon la revendication 16, **caractérisée par le fait que** les polysiloxanes à groupements aminés sont choisis parmi les amodiméthicones ou les triméthylsilylamodiméthicones.

18. Composition selon l'une quelconque des revendications 1 à 17, **caractérisée par le fait qu'**elle comprend en outre au moins un agent bénéfique choisi parmi les esters d'acides carboxyliques en C1-C30 et de d'alcools mono ou polyhydroxylés en C1-C30, les huiles végétales, animales, minérales ou de synthèse, les cires, les céramides, les pseudocéramides.

19. Composition selon la revendication 18, **caractérisée par le fait que** les esters d'acides carboxyliques en C1-C30 et de d'alcools mono ou polyhydroxylés en C1-C30 sont choisis parmi le myristate d'isopropyle, l'isononanoate d'isononyle, et leurs mélanges.

20. Composition selon la revendication 18, **caractérisée par le fait que** les huiles végétales sont choisies parmi l'huile d'avocat, l'huile d'olive et leurs mélanges.

21. Composition selon l'une quelconque des revendications précédentes, **caractérisée en ce que** le phosphate d'amidon est présent à une concentration allant de 0,01 % à 20 % en poids par rapport au poids total de la composition, de préférence de 0,05 % à 15 % en poids.

22. Composition selon l'une quelconque des revendications précédentes, **caractérisée en ce que** le polymère cationique est présent à une concentration allant de 0,001 % à 20 % en poids par rapport au poids total de la composition, de préférence de 0,01 % à 10 % en poids.

23. Composition selon l'une quelconque des revendications 12 à 22, **caractérisée en ce que** ladite silicone est présente à une concentration allant de 0,001 % à 20 % en poids par rapport au poids total de la composition, de préférence de 0,01 % à 10 % en poids.

24. Composition selon l'une quelconque des revendications 17 à 23, **caractérisée en ce que** l'agent bénéfique pour les cheveux est présent à une concentration allant de 0,001 % à 20 % en poids par rapport au poids total de la composition, de préférence de 0,01 % à 10 % en poids.

25. Composition selon l'une des revendications précédentes, **caractérisée par** le fait les tensioactifs cationiques sont choisis parmi les sels de béhényltriméthylammonium, les sels de stéaramidopropyldiméthyl (myristyl acétate) ammonium, les sels de palmitylamidopropyl triméthyl ammonium, le Quaternium-27 ou le Quaternium-83.

26. Composition selon l'une quelconque des revendications précédentes, **caractérisée par le fait que** le ou les tensioactifs sont présents à une concentration allant de 0,01% à 50% en poids, de préférence allant de 0,1% à 40% en poids, et encore plus préférentiellement allant de 0,5% à 30% en poids, par rapport au poids total de la composition.

27. Composition selon l'une quelconque des revendications 1 à 26, **caractérisée par le fait qu'**elle comprend au moins un tensioactif cationique et au moins une silicone.

28. Composition selon l'une quelconque des revendications précédentes, **caractérisée par le fait qu'**elle se présente sous forme de shampooing, d'après-shampooing, de composition pour la permanente, le défrisage, la coloration ou la décoloration des cheveux, de composition à rincer à appliquer entre les deux étapes d'une permanente ou d'un défrisage, de composition lavantes pour le corps.

29. Composition selon l'une quelconque des revendications précédentes, **caractérisée par le fait qu'**elle se présente sous forme d'après-shampooing, notamment d'après-shampooing à rincer.

30. Utilisation d'une composition telle que définie dans l'une quelconque des revendications 1 à 29, pour le lavage ou pour le soin des matières kératiniques.

31. Procédé de traitement des matières kératiniques, telles que les cheveux, **caractérisé en ce qu'**il consiste à appliquer sur lesdites matières une composition cosmétique selon l'une des revendications 1 à 29, puis à effectuer éventuellement un rinçage à l'eau.

## Patentansprüche

1. Kosmetische Zusammensetzung, **dadurch gekennzeichnet, dass** sie in einem kosmetisch unbedenklichen Medium Folgendes umfasst:
- mindestens ein Tensid, das aus anionischen, nichtionischen, amphoteren und kationischen Tensiden und Mischungen davon ausgewählt ist;
- mindestens ein Stärkephosphat und
- mindestens ein kationisches Nichtsilikon-Polymer, ausgewählt aus:
(2) kationischen Cellulosen,
(5) Polyaminoamidderivaten, die sich aus der Kondensation von Polyalkylenpolyaminen mit Polycarbonsäuren und einer anschließenden Alkylierung mit bifunktionellen Mitteln ergeben,
(7) Cyclopolymeren von Alkyldiallylamin oder Dialkyldiallylammonium mit Ausnahme des Copolymers von Diallyldimethylammoniumchlorid und Acrylamid,
(8) quaternären Diammoniumpolymeren mit Wiederholungseinheiten der Formel: 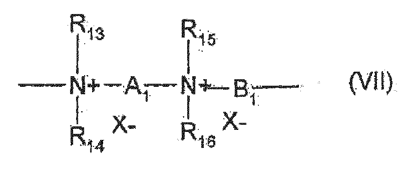 worin:
R₁₃, R₁₄, R₁₅ und R₁₆ gleich oder verschieden sind und für aliphatische, alicyclische oder arylaliphatische Reste mit 1 bis 20 Kohlenstoffatomen oder niedere hydroxyalkylaliphatische Reste stehen oder auch R₁₃, R₁₄, R₁₅ und R₁₆ zusammen oder separat mit den Stickstoffatomen, an die sie gebunden sind, Heterocyclen bilden, die gegebenenfalls neben dem Stickstoff ein zweites Heteroatom enthalten, oder auch R₁₃, R₁₄, R₁₅ und R₁₆ für einen linearen oder verzweigten C₁-C₆-Alkylrest stehen, der durch eine Nitril-, Ester-, Acyl-, Amid- oder -CO-O-R₁₇-D- oder -CO-NH-R₁₇-D-Gruppe substituiert ist, wobei R₁₇ für ein Alkylen steht und D für eine quaternäre Ammoniumgruppe steht;
A₁ und B₁ für Polymethylengruppen mit 2 bis 20 Kohlenstoffatomen stehen, die linear oder verzweigt und gesättigt oder ungesättigt sein können und an die Hauptkette gebunden oder in die Hauptkette eingeschoben einen oder mehrere aromatische Ringe oder ein oder mehrere Sauerstoff- oder Schwefelatome oder Sulfoxid-, Sulfon-, Disulfid-, Amino-, Alkylamino-, Hydroxyl-, quaternäre Ammonium-, Ureido-, Amid- oder Estergruppen enthalten können, und
X⁻ für ein Anion, das sich von einer anorganischen oder organischen Säure ableitet, steht;
A₁, R₁₃ und R₁₅ mit den beiden Stickstoffatomen, an die sie gebunden sind, einen Piperazinring bilden können; außerdem B₁ dann, wenn A₁ für einen linearen oder verzweigten, gesättigten oder ungesättigten, Alkylen- oder Hydroxyalkylenrest steht, auch für eine (CH₂)ₙ-CO-D-OC-(CH₂)ₙ-Gruppe stehen kann,
worin D für:
a) einen Glykolrest der Formel: -O-Z-O-, worin Z für einen linearen oder verzweigten Kohlenwasserstoffrest oder eine Gruppe, die einer der folgenden Formeln entspricht, steht:
-(CH₂-CH₂-O)ₓ-CH₂-CH₂-
- [CH₂-CH(CH₃)-O]_{y}-CH₂-CH(CH₃) -
wobei x und y für eine ganze Zahl von 1 bis 4 stehen und einen definierten und einzigartigen Polymerisationsgrad wiedergeben oder für eine beliebige Zahl von 1 bis 4 stehen und einen durchschnittlichen Polymerisationsgrad wiedergeben;
b) einen bissekundären Diaminrest wie ein Piperazinderivat steht;
c) einen bisprimären Diaminrest der Formel: -NH-Y-NH-, wobei Y für einen linearen oder verzweigten Kohlenwasserstoffrest oder auch den zweiwertigen Rest
-CH₂-CH₂-S-S-CH₂-CH₂-
steht;
d) eine Ureylengruppe der Formel: -NH-CO-NHsteht;
X⁻ vorzugsweise für ein einwertiges anorganisches oder organisches Anion steht;
(10) quaternären Vinylpyrrolidon- und Vinylimidazolpolymeren;
(12) vernetzten oder nicht vernetzten Polymeren von Methacryloyloxy(C₁-C₄)alkyltri(C₁-C₄)alkyl-ammoniumsalzen.

2. Zusammensetzung nach Anspruch 1, **dadurch gekennzeichnet, dass** es sich bei dem Stärkephosphat um einen Ester von Phosphorverbindungen und mindestens einer Stärke handelt.

3. Zusammensetzung nach dem vorhergehenden Anspruch, **dadurch gekennzeichnet, dass** die Phosphorverbindung aus Natriumtripolyphosphat, Natriumorthophosphat, Phosphoroxidchlorid und Natriumtrimetaphosphat ausgewählt ist.

4. Zusammensetzung nach einem der Ansprüche 1 bis 3, **dadurch gekennzeichnet, dass** die Stärken aus Mais-, Reis-, Maniok-, Tapioka-, Gersten-, Kartoffel-, Weizen-, Sorghum- und Erbsenstärken ausgewählt ist.

5. Zusammensetzung nach einem der Ansprüche 1 bis 4, **dadurch gekennzeichnet, dass** die Stärke C1-C6-hydroxyalkyliert oder C1-C6-acyliert ist.

6. Zusammensetzung nach einem der Ansprüche 1 bis 5, **dadurch gekennzeichnet, dass** das Stärkephosphat aus Monostärkephosphaten, Distärkephosphaten, Tristärkephosphaten und Mischungen davon ausgewählt ist.

7. Zusammensetzung nach Anspruch 6, **dadurch gekennzeichnet, dass** das Stärkephosphat aus Distärkephosphaten ausgewählt ist.

8. Zusammensetzung nach einem der Ansprüche 1 bis 5, **dadurch gekennzeichnet, dass** das Stärkephosphat aus verkleisterten hydroxypropylierten Dimaniokstärkephosphaten, verkleisterten acetylierten Dimaniokstärkephosphaten und verkleisterten Dimaniokstärkephosphaten ausgewählt ist.

9. Zusammensetzung nach einem der vorhergehenden Ansprüche, **dadurch gekennzeichnet, dass** das kationische Nichtsilikon-Polymer ausgewählt ist aus:
(2) Celluloseetherderivaten mit quaternären Ammoniumgruppen, kationischen Cellulosecopolymeren oder Cellulosederivaten, die mit einem wasserlöslichen Ammonium-Monomer gepfropft sind;
(5) Adipinsäure-Dialkylaminohydroxyalkyldialkylentriamin-Polymere, wobei der Alkylrest 1 bis 4 Kohlenstoffatome enthält;
(7) Homopolymeren oder Copolymeren, enthaltend als Hauptbestandteil der Kette Einheiten der Formeln (VI) oder (VI'): 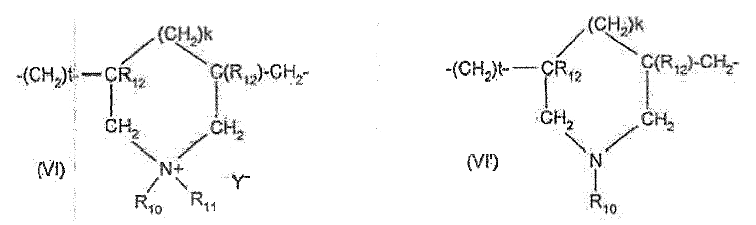 worin k und t gleich 0 oder 1 sind, die Summe k + t gleich 1 ist; R₁₂ für ein Wasserstoffatom oder einen Methylrest steht; R₁₀ und R₁₁ unabhängig voneinander für eine Alkylgruppe mit 1 bis 22 Kohlenstoffatomen, eine Hydroxyalkylgruppe, in der die Alkylgruppe vorzugsweise 1 bis 5 Kohlenstoffatome aufweist, oder eine Amidoniederalkyl(C1-C4)-gruppe stehen oder R₁₀ und R₁₁ zusammen mit dem Stickstoffatom, an das sie gebunden sind, für heterocyclische Gruppen, wie Piperidinyl oder Morpholinyl, stehen können; Y⁻ ein Anion wie Bromid, Chlorid, Acetat, Borat, Citrat, Tartrat, Hydrogensulfat, Hydrogensulfit, Sulfat oder Phosphat bedeutet,
mit Ausnahme des Copolymers von Diallyldimethylammoniumchlorid und Acrylamid;
(8) quaternären Diammoniumpolymeren mit Wiederholungseinheiten der Formel: 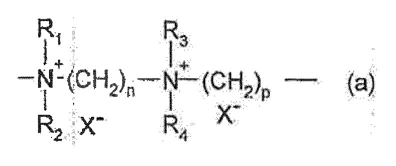 worin:
R₁, R₂, R₃ und R₄ gleich oder verschieden sind und für einen Alkyl- oder Hydroxyalkylrest mit ungefähr 1 bis 4 Kohlenstoffatomen stehen, n und p ganze Zahlen von ungefähr 2 bis 20 sind und X⁻ ein Anion, das sich von einer anorganischen oder organischen Säure ableitet, ist;
(12) Polymeren, erhalten durch Homopolymerisation von Dimethylaminoethylmethacrylat, das mit Methylchlorid quaternisiert ist, oder durch Copolymerisation von Acrylamid oder Methacrylamiden mit Dimethylaminoethylmethacrylat, das mit Methylchlorid quaternisiert ist, wobei sich an die Homo- bzw. Copolymerisation eine Vernetzung mit einer olefinisch ungesättigten Verbindung, insbesondere Methylenbisacrylamid, anschließt.

10. Zusammensetzung nach einem der vorhergehenden Ansprüche, **dadurch gekennzeichnet, dass** das kationische Nichtsilikon-Polymer aus Homopolymeren von Diallyldimethylammoniumchlorid ausgewählt ist.

11. Zusammensetzung nach einem der Ansprüche 1 bis 9, **dadurch gekennzeichnet, dass** die kationischen Cellulosen aus mit einem durch eine Trimethylammoniumgruppe substituierten Epoxid umgesetzten Hydroxyethylcellulosen ausgewählt sind.

12. Zusammensetzung nach einem der Ansprüche 1 bis 11, **dadurch gekennzeichnet, dass** sie außerdem mindestens ein Silikon umfasst.

13. Zusammensetzung nach Anspruch 12, **dadurch gekennzeichnet, dass** die Silikone aus Polyorganosiloxanen, die in der Zusammensetzung unlöslich sind, ausgewählt sind.

14. Zusammensetzung nach einem der Ansprüche 12 oder 13, **dadurch gekennzeichnet, dass** es sich bei den Silikonen um nichtflüchtige Polyorganosiloxane, die aus Polyalkylsiloxanen, Polyarylsiloxanen, Polyalkylarylsiloxanen, Silikongummen und -harzen, mit organofunktionellen Gruppen modifizierten Polyorganosiloxanen sowie Mischungen davon ausgewählt sind, handelt.

15. Zusammensetzung nach Anspruch 14, **dadurch gekennzeichnet, dass**:
(a) die Polyalkylsiloxane ausgewählt sind aus:
- Polydimethylsiloxanen mit Trimethylsilyl-Endgruppen;
- Polydimethylsiloxane mit Dimethylsilanol-Endgruppen;
- Poly(C₁-C₂₀)alkylsiloxanen;
(b) die Polyalkylarylsiloxane ausgewählt sind aus:
- Polydimethylmethylphenylsiloxanen und linearen und/oder verzweigten Polydimethyldiphenylsiloxanen mit einer Viskosität von 1.10⁻⁵ bis 5.10⁻² m²/s bei 25°C;
(c) die Silikongummen ausgewählt sind aus Polydiorganosiloxanen mit zahlenmittleren Molekulargewichten von 200.000 bis 1.000.000, die alleine oder in Form einer Mischung in einem Lösungsmittel verwendet werden;
(d) die Harze ausgewählt sind aus Harzen, die aus R₃SiO_{1/2}-, R₂SiO_{2/2}-, RSiO_{3/2}-, SiO_{4/2}-Einheiten bestehen,
wobei R für eine Kohlenwasserstoffgruppe mit 1 bis 16 Kohlenstoffatomen oder eine Phenylgruppe steht;
(e) die organomodifizierten Silikone ausgewählt sind aus Silikonen, die in ihrer Struktur eine oder mehrere organofunktionelle Gruppen, die über einen Kohlenwasserstoffrest gebunden sind, enthalten.

16. Zusammensetzung nach einem der Ansprüche 12 bis 15, **dadurch gekennzeichnet, dass** die Silikone aus Polyalkylsiloxanen mit Trimethylsilyl-Endgruppen, Polyalkylsiloxanen mit Dimethylsilanol-Endgruppen, Polyalkylarylsiloxanen, Mischungen der beiden PDMS, die aus einem Gummi und einem Öl mit verschiedenen Viskositäten, Mischungen von Organosiloxanen und cyclischen Silikonen, Organopolysiloxanharzen und Polysiloxanen mit Aminogruppen ausgewählt sind.

17. Zusammensetzung nach Anspruch 16, **dadurch gekennzeichnet, dass** die Polysiloxane mit Aminogruppen aus Amodimethiconen und Trimethylsilylamodimethiconen ausgewählt sind.

18. Zusammensetzung nach einem der Ansprüche 1 bis 17, **dadurch gekennzeichnet, dass** sie außerdem mindestens ein Vorteilsmittel, das aus Estern von C1-C30-Carbonsäuren und ein- oder mehrwertigen C1-C30-Alkoholen, pflanzlichen, tierischen, mineralischen oder synthetischen Ölen, Wachsen, Ceramiden und Pseudoceramiden ausgewählt ist, umfasst.

19. Zusammensetzung nach Anspruch 18, **dadurch gekennzeichnet, dass** die Ester von C1-C30-Carbonsäuren und ein- oder mehrwertigen C1-C30-Alkoholen aus Isopropylmyristat, Isononylisononanoat und Mischungen davon ausgewählt sind.

20. Zusammensetzung nach Anspruch 18, **dadurch gekennzeichnet, dass** die pflanzlichen Öle aus Avocadoöl, Olivenöl und Mischungen davon ausgewählt sind.

21. Zusammensetzung nach einem der vorhergehenden Ansprüche, **dadurch gekennzeichnet, dass** das Stärkephosphat in einer Konzentration von 0,01 bis 20 Gew.-%, bezogen auf das Gesamtgewicht der Zusammensetzung, vorzugsweise 0,05 bis 15 Gew.-%, vorliegt.

22. Zusammensetzung nach einem der vorhergehenden Ansprüche, **dadurch gekennzeichnet, dass** das kationische Polymer in einer Konzentration von 0,001 bis 20 Gew.-%, bezogen auf das Gesamtgewicht der Zusammensetzung, vorzugsweise 0,01 bis 10 Gew.-%, vorliegt.

23. Zusammensetzung nach einem der Ansprüche 12 bis 22, **dadurch gekennzeichnet, dass** das Silikon in einer Konzentration von 0,001 bis 20 Gew.-%, bezogen auf das Gesamtgewicht der Zusammensetzung, vorzugsweise 0,01 bis 10 Gew.-%, vorliegt.

24. Zusammensetzung nach einem der Ansprüche 17 bis 23, **dadurch gekennzeichnet, dass** das Vorteilsmittel für die Haare in einer Konzentration von 0,001 bis 20 Gew.-%, bezogen auf das Gesamtgewicht der Zusammensetzung, vorzugsweise 0,01 bis 10 Gew.-%, vorliegt.

25. Zusammensetzung nach einem der vorhergehenden Ansprüche, **dadurch gekennzeichnet, dass** die kationischen Tenside aus Behenyltrimethylammoniumsalzen, Stearamidopropyldimethyl(myristylacetat)-ammoniumsalzen, Palmitylamidopropyltrimethylammoniumsalzen, Quaternium-27 oder Quaternium-83 ausgewählt sind.

26. Zusammensetzung nach einem der vorhergehenden Ansprüche, **dadurch gekennzeichnet, dass** das bzw. die Tenside in einer Konzentration von 0,01 bis 50 Gew.-%, vorzugsweise 0,1 bis 40 Gew.-% und noch weiter bevorzugt 0,5 bis 30 Gew.-% bezogen auf das Gesamtgewicht der Zusammensetzung, vorliegen.

27. Zusammensetzung nach einem der Ansprüche 1 bis 26, **dadurch gekennzeichnet, dass** sie mindestens ein kationisches Tensid und mindestens ein Silikon umfasst.

28. Zusammensetzung nach einem der vorhergehenden Ansprüche, **dadurch gekennzeichnet, dass** sie in Form eines Shampoos, einer Haarpflegespülung, einer Zusammensetzung für eine Dauerwelle, eine Haarglättung, eine Färbung oder Bleichung der Haare, einer Wash-out-Zusammensetzung zur Anwendung zwischen den beiden Schritten einer Dauerwelle oder Haarglättung oder Waschzusammensetzung für den Körper vorliegt.

29. Zusammensetzung nach einem der vorhergehenden Ansprüche, **dadurch gekennzeichnet, dass** sie in Form einer Haarpflegespülung, insbesondere einer Washout-Haarpflegespülung vorliegt.

30. Verwendung einer Zusammensetzung gemäß einem der Ansprüche 1 bis 29 zum Waschen oder Pflegen von Keratinmaterialien.

31. Verfahren zur Behandlung von Keratinmaterialien, wie den Haaren, **dadurch gekennzeichnet, dass** es darin besteht, dass man auf die Materialien eine wirksame Menge einer kosmetischen Zusammensetzung nach einem der Ansprüche 1 bis 29 aufbringt und danach gegebenenfalls eine Spülung mit Wasser durchführt.

## Claims

1. Cosmetic composition, **characterized in that** it comprises, in a cosmetically acceptable medium:
- at least one surfactant chosen from anionic, nonionic, amphoteric and cationic surfactants, and mixtures thereof;
- at least one starch phosphate and
- at least one nonsilicone cationic polymer chosen from:
(2) cationic celluloses,
(5) polyaminoamide derivatives resulting from the condensation of polyalkylenepolyamines with polycarboxylic acids, followed by an alkylation with bifunctional agents,
(7) cyclopolymers of alkyldiallylamine or of dialkyldiallylammonium, except for the copolymer of diallyldimethylammonium chloride and acrylamide,
(8) quaternary diammonium polymers comprising repeat units corresponding to the formula: 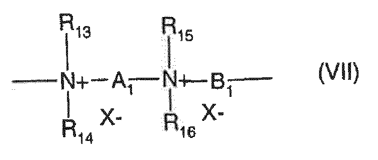 in which:
R₁₃, R₁₄, R₁₅ and R₁₆, which are identical or different, represent aliphatic, alicyclic or arylaliphatic radicals containing from 1 to 20 carbon atoms or lower hydroxyalkyl aliphatic radicals, or else R₁₃, R₁₄, R₁₅ and R₁₆, together or separately, form, with the nitrogen atoms to which they are attached, heterocyclic rings optionally containing a second heteroatom other than nitrogen, or else R₁₃, R₁₄, R₁₅ and R₁₆ represent a linear or branched C₁-C₆ alkyl radical substituted by a nitrile, ester, acyl, amide or -CO-O-R₁₇-D or -CO-NH-R₁₇-D group where R₁₇ is an alkylene and D a quaternary ammonium group;
A₁ and B₁ represent polymethylene groups containing from 2 to 20 carbon atoms which may be linear or branched, saturated or unsaturated and which may contain, bonded to or inserted into the main chain, one or more aromatic rings, or one or more oxygen or sulfur atoms or sulfoxide, sulfone, disulfide, amino, alkylamino, hydroxyl, quaternary ammonium, ureido, amide or ester groups, and
X- denotes an anion derived from an inorganic or organic acid;
A₁, R₁₃ and R₁₅, with the two nitrogen atoms to which they are attached, may form a piperazine ring; in addition if A₁ denotes a saturated or unsaturated, linear or branched alkylene or hydroxyalkylene radical, B₁ may also denote a group (CH₂)ₙ-CO-D-OC- (CH₂)ₙ-
in which D denotes:
a) a glycol residue of formula: -O-Z-O-, where Z denotes a linear or branched hydrocarbon radical or a group corresponding to one of the following formulae:
-(CH₂-CH₂-O)ₓ-CH₂-CH₂-
- [CH₂-CH(CH₃)-O]_{y}-CH₂-CH(CH₃) -
where x and y denote an integer from 1 to 4, representing a defined and unique degree of polymerization or any number from 1 to 4 representing a mean degree of polymerization;
b) a disecondary diamine residue such as a piperazine derivative;
c) a diprimary diamine residue of formula: -NH-Y-NH-, where Y denotes a linear or branched hydrocarbon radical or else the divalent radical
-CH₂-CH₂-S-S-CH₂-CH₂-;
d) a ureylene group of formula: -NH-CO-NH-;
(10) quaternary vinylpyrrolidone and vinylimidazole polymers,
(12) crosslinked or noncrosslinked polymers of methacryloyloxy(C₁-C₄ alkyl)tri(C₁-C₄ alkyl)ammonium salts.

2. Composition according to Claim 1, **characterized in that** the starch phosphate is an ester of phosphorus-containing compounds and at least one starch.

3. Composition according to the preceding claim, **characterized in that** the phosphorus-containing compound is chosen from sodium tripolyphosphate, sodium orthophosphate, phosphorus oxychloride and sodium trimetaphosphate.

4. Composition according to any one of Claims 1 to 3, **characterized in that** the starches are chosen from maize, rice, cassava, tapioca, barley, potato, wheat, sorghum and pea starches.

5. Composition according to any one of Claims 1 to 4, **characterized in that** the starch is C₁-C₆ hydroxyalkylated or C₁-C₆ acylated.

6. Composition according to any one of Claims 1 to 5, **characterized in that** the starch phosphate is chosen from monostarch phosphates, distarch phosphates, tristarch phosphates and mixtures thereof.

7. Composition according to Claim 6, **characterized in that** the starch phosphate is chosen from distarch phosphates.

8. Composition according to any one of Claims 1 to 5, **characterized in that** the starch phosphate is chosen from gelatinized hydroxypropylated cassava distarch phosphates, gelatinized acetylated cassava distarch phosphates and gelatinized cassava distarch phosphates.

9. Composition according to any one of the preceding claims, **characterized in that** the nonsilicone cationic polymer is chosen from:
(2) cellulose ether derivatives comprising quaternary ammonium groups, the cationic cellulose copolymers or the cellulose derivatives grafted with a water-soluble ammonium monomer;
(5) the adipic acid-dialkylaminohydroxyalkyldialkylenetriamine polymers in which the alkyl radical contains from 1 to 4 carbon atoms;
(7) the homopolymers or copolymers comprising, as main constituent of the chain, units corresponding to the formulae (VI) or (VI') : 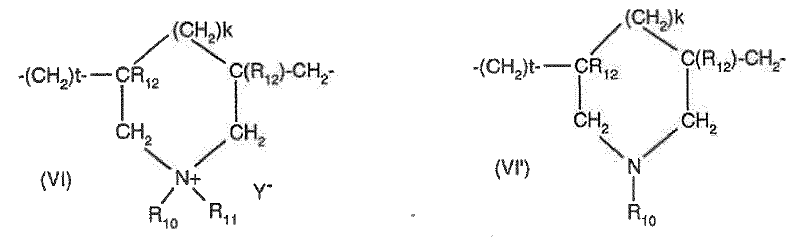 in which k and t are equal to 0 or 1, the sum k + t being equal to 1; R₁₂ denotes a hydrogen atom or a methyl radical; R₁₀ and R₁₁, independently of each other, denote an alkyl group containing from 1 to 22 carbon atoms, a hydroxyalkyl group in which the alkyl group preferably has 1 to 5 carbon atoms, or a lower (C₁-C₄)amidoalkyl group or R₁₀ and R₁₁ may denote, jointly with the nitrogen atom to which they are attached, heterocyclic groups such as piperidinyl or morpholinyl; Y⁻ is an anion such as bromide, chloride, acetate, borate, citrate, tartrate, bisulphate, bisulphite, sulphate or phosphate,
except for the copolymer of diallyl-dimethylammonium chloride and acrylamide;
(8) the quaternary diammonium polymers consisting of repeat units corresponding to the formula: 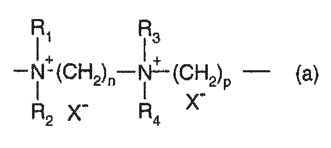 in which R₁, R₂, R₃ and R₄, which are identical or different, denote an alkyl or hydroxyalkyl radical having from 1 to 4 carbon atoms approximately, n and p are integers varying from 2 to 20 approximately and X⁻ is an anion derived from an inorganic or organic acid;
(12) the polymers obtained by homopolymerization of dimethylaminoethyl methacrylate quaternized with methyl chloride, or by copolymerization of acrylamide or methacrylamide with dimethylaminoethyl methacrylate quaternized with methyl chloride, the homo- or copolymerization being followed by crosslinking with a compound containing olefinic unsaturation, in particular methylenebisacrylamide.

10. Composition according to one of the preceding claims, **characterized in that** the nonsilicone cationic polymer is chosen from homopolymers of diallyldimethylammonium chloride.

11. Composition according to one of Claims 1 to 9, **characterized in that** the cationic celluloses are chosen from hydroxyethylcelluloses which have reacted with an epoxide substituted with a trimethylammonium group.

12. Composition according to any one of Claims 1 to 11, **characterized in that** it comprises, in addition, at least one silicone.

13. Composition according to Claim 12, **characterized in that** the silicones are chosen from polyorganosiloxanes insoluble in the composition.

14. Composition according to either of Claims 12 and 13, **characterized in that** the silicones are nonvolatile polyorganosiloxanes chosen from polyalkylsiloxanes, polyarylsiloxanes, polyalkylarylsiloxanes, silicone gums and resins, polyorganosiloxanes modified by organofunctional groups, and mixtures thereof.

15. Composition according to Claim 14, **characterized in that**:
(a) the polyalkylsiloxanes are chosen from:
- polydimethylsiloxanes with trimethylsilyl terminal groups;
- polydimethylsiloxanes with dimethylsilanol terminal groups;
- poly(C₁-C₂₀)alkylsiloxanes;
(b) the polyalkylarylsiloxanes are chosen from:
- polydimethylmethylphenylsiloxanes, polydimethyldiphenylsiloxanes which are linear and/or branched and have a viscosity ranging from 1 x 10⁻⁵ to 5 × 10⁻² m²/s at 25°C;
(c) the silicone gums are chosen from polydiorganosiloxanes having number-average molecular masses ranging from 200,000 to 1,000,000, used alone or in the form of a mixture in a solvent;
(d) the resins are chosen from the resins consisting of units: R₃ Si O_{1/2}, R₂ Si O_{2/2}, R Si O_{3/2}, Si O_{4/2} in which R represents a hydrocarbon group having from 1 to 16 carbon atoms or a phenyl group;
(e) the organomodified silicones are chosen from silicones comprising in their structure one or more organofunctional groups attached via a hydrocarbon radical.

16. Composition according to any one of Claims 12 to 15, **characterized in that** the silicones are chosen from polyalkylsiloxanes with trimethylsilyl terminal groups, polyalkylsiloxanes with dimethylsilanol terminal groups, polyalkylarylsiloxanes, mixtures of two PDMSs consisting of a gum and an oil with different viscosities, mixtures of cyclic silicones and organosiloxanes, organopolysiloxane resins, and polysiloxanes containing amino groups.

17. Composition according to Claim 16, **characterized in that** the polysiloxanes containg amino groups are chosen from amodimethicones or tromethylsilylamodimethicones.

18. Composition according to any one of Claims 1 to 17, **characterized in that** it comprises, in addition, at least one beneficial agent chosen from esters of C₁-C₃₀ carboxylic acids and C₁-C₃₀ mono- or polyhydroxylated alcohols, vegetable, animal, mineral or synthetic oils, waxes, ceramides and pseudoceramides.

19. Composition according to Claim 18, **characterized in that** the esters of C₁-C₃₀ carboxylic acids and C₁-C₃₀ mono- or polyhydroxylated alcohols are chosen from isopropyl myristate, isononyl isononanoate and mixtures thereof.

20. Composition according to Claim 18, **characterized in that** the vegetable oils are chosen from avocado oil, olive oil and mixtures thereof.

21. Composition according to any one of the preceding claims, **characterized in that** the starch phosphate is present at a concentration ranging from 0.01% to 20% by weight relative to the total weight of the composition, preferably from 0.05% to 15% by weight.

22. Composition according to any one of the preceding claims, **characterized in that** the cationic polymer is present at a concentration ranging from 0.001% to 20% by weight relative to the total weight of the composition, preferably from 0.01% to 10% by weight.

23. Composition according to any one of Claims 12 to 22, **characterized in that** the said silicone is present at a concentration ranging from 0.001% to 20% by weight relative to the total weight of the composition, preferably from 0.01% to 10% by weight.

24. Composition according to any one of Claims 17 to 23, **characterized in that** the agent beneficial to the hair is present at a concentration ranging from 0.001% to 20% by weight relative to the total weight of the composition, preferably from 0.01% to 10% by weight.

25. Composition according to one of the preceding claims, **characterized in that** the cationic surfactants are chosen from behenyltrimethylammonium salts, stearamidopropyldimethyl(myristylacetate)ammonium salts, palmitylamidopropyltrimethylammonium salts, Quaternium-27 or Quaternium-83.

26. Composition according to any one of the preceding claims, **characterized in that** the surfactant(s) are present at a concentration ranging from 0.01% to 50% by weight, preferably ranging from 0.1% to 40% by weight, and more preferably still ranging from 0.5% to 30% by weight, relative to the total weight of the composition.

27. Composition according to any one of Claims 1 to 26, **characterized in that** it additionally comprises at least one cationic surfactant and at least one silicone.

28. Composition according to any one of the preceding claims, **characterized in that** it is provided in the form of a shampoo, an after-shampoo, a composition for permanent waving, straightening, dyeing or bleaching the hair, a rinse-off composition to be applied between the two stages of a permanent waving or hair straightening, or a washing composition for the body.

29. Composion according to any one of the preceding claims, **characterized in that** it is in the form of an after-shampoo, in particular of an after-shampoo to be rinsed off.

30. Use of a composition as defined in any one of Claims 1 to 29, for the washing or for the care of keratinous materials.

31. Method for treating keratinous materials, such as the hair, **characterized in that** it consists in applying to the said materials a cosmetic composition according to one of Claims 1 to 29, and then in optionally rinsing with water.
